# EUROPÄISCHE PATENTSCHRIFT

(11) **EP 0 995 396 B1**
(45) Veröffentlichungstag und Bekanntmachung des Hinweises auf die Patenterteilung: **06.05.2004**
(21) Anmeldenummer: 99121172.3
(22) Anmeldetag: 22.10.1999
(51) Int. Cl.: A61B 5/0452

(54) **Elektrokardiogramm-Anordnung**
Electrocardiographic system
Dispositif d'électrocardiographie

(30) Priorität: 23.10.1998 DE 19849034
(43) Veröffentlichungstag der Anmeldung: 26.04.2000
(73) Patentinhaber: GE Medical Systems Information Technologies GmbH, 79111 Freiburg i. Br. (DE)
(72) Erfinder: Kaiser, Willi, 79312 Emmendingen (DE); Findeis, Martin, 79111 Freiburg (DE)
(74) Vertreter: Müller - Hoffmann & Partner

(56) Entgegenhaltungen:
- US-A- 4 583 553
- US-A- 4 860 762
- COKER C W ET AL: "EKG ELECTRODE ARTIFACT REJECTION CIRCUIT" IBM TECHNICAL DISCLOSURE BULLETIN,US,IBM CORP. NEW YORK, Bd. 23, Nr. 7B, Dezember 1980 (1980-12), Seite 3493-3497 XP002029529 ISSN: 0018-8689
- ARMINGTON R M ET AL: "OPTIMIZING THE UTILIZATION OF MULTI-LEAD ECG DATA FOR ACCURATE QRS DETECTION" PROCEEDINGS OF THE COMPUTERS IN CARDIOLOGY MEETING,US,WASHINGTON, IEEE COMP. SOC. PRESS, Bd. MEETING 13, Oktober 1986 (1986-10), Seite 259-262 XP002053557

## Beschreibung

Die vorliegende Erfindung betrifft eine Elektrokardiogramm-(EKG-)Anordnung mit m-(m > 2)Ableitungskanälen zur Lokalisation von QRS-Komplexen in einem Elektrokardiogramm und mit einer zentralen Logikeinheit.

Bei EKG-Untersuchungen werden bekanntlich immer häufiger Elektrokardiogramm-Anordnungen mit mehreren Ableitungskanälen eingesetzt, die üblicherweise an Armen und Beinen (Gliedmaßenableitung) und an der Brustwand (Brustwandableitung) angelegt werden. Am verbreitetsten sind dabei Elektrokardiogramm-Anordnungen, die über zwölf Ableitungskanäle verfügen. Es gibt jedoch auch Elektrokardiogramm-Anordnungen, bei denen mehr oder weniger als zwölf Ableitungskanäle zum Einsatz gelangen.

Beispielsweise für die Herzfrequenzermittlung oder die Arrhythmieanalyse ergeben sich durch die Redundanz der mehreren Ableitungskanäle Vorteile, wenn diese Redundanz dazu verwendet wird, den Einfluß von Störungen bzw. Artefakten in den durch die Elektrokardiogramm-Anordnung gewonnenen Ergebnis zu vermindern bzw. zu eliminieren.

Bisherige Elektrokardiogramm-Anordnungen nutzen die Redundanz der mehreren Ableitungskanäle aus und verknüpfen die einzelnen Ableitungskanal-Signale, indem von diesen die erste mathematische Ableitung gebildet wird. Die einzelnen mathematischen Ableitungen werden quadriert und sodann über alle Ableitungskanäle addiert. Aus dem auf diese Weise erhaltenen Ergebnis wird sodann die Wurzel gezogen. Das auf diese Weise gewonnene Signal hat den Vorteil, daß in ihm im Vergleich zu den einzelnen ursprünglichen Signalen das Verhältnis von Nutzsignalanteil zu Störsignalanteil verbessert ist, was letzten Endes bedeutet, daß die QRS-Komplexe besser lokalisiert werden können.

Anstelle der Quadrierung und des Wurzelziehens wird gelegentlich auch lediglich der Betrag der durch die erste mathematische Ableitung erhaltenen Signale gebildet, woran sich ein Addieren der für die einzelnen Ableitungskanäle gewonnenen Beträge anschließt.

Bei beiden Elektrokardiogramm-Anordnungen wird jedenfalls auf diese Weise schließlich ein einkanaliges Signal erhalten, das einer Auswertung hinsichtlich der QRS-Komplexe unterworfen wird.

Die in dieser Weise aufgebauten herkömmlichen Elektrokardiogramm-Anordnungen liefern zuverlässige Ergebnisse, wenn der Störgrad in allen Ableitungskanälen ähnlich groß ist. Sind aber einzelne Ableitungskanäle wesentlich stärker gestört als andere Ableitungskanäle, was häufig beispielsweise bei der Aufnahme eines Belastungs-Elektrokardiogrammes der Fall ist, ist es zweckmäßig, die Signale der stark gestörten Ableitungskanäle von einer Weiterverarbeitung auszuschließen.

Hierzu gibt es bereits Elektrokardiogramm-Anordnungen, die zwar in der oben aufgezeigten Weise aufgebaut sind, jedoch nur Signale von Ableitungskanälen verwenden, die erfahrungsgemäß weniger Artefakte haben und ungefähr orthogonal zueinander stehen. Durch die Orthogonalität der Signale von Ableitungskanälen wird nämlich erreicht, daß zumindest in einem Signal dann die normalen QRS-Komplexe oder ventrikulären Extrasystolen gut repräsentant sind. Nachteilhaft an einem derartigen Vorgehen ist aber, daß infolge der Forderung, orthogonal zueinander stehende Signale auszuwerten, auch Signale von Ableitungskanälen verwendet werden müssen, die stärker gestört sind als andere Signale, so daß eine derart aufgebaute Elektrokardiogramm-Anordnung letzten Endes auch nur bedingt gute Ergebnisse zu liefern vermag.

Elektrokardiogramm-Anordnungen der oben beschriebenen Art sind bekannt aus Jan A. Kors, Jan L. Talmon und Jan H. Van Bemmel: "Multilead ECG Analysis" in "Computers And Biomedical Research 19, Seiten 28 - 46, 1986 und Christian R. Brohet, Christiane Erwael-Barchy, Robert Fesler und Lucien A. Brasseur: "Arrhythmia Analysis by the Louvain VCG Program" in "Computers in Cardiology", Seiten 47 - 51. 1981.

Weiterhin sind in US 4,987,901 eine spezielle Anordnung der Elektroden auf der Körperoberfläche und ein auf dieser speziellen Elektrodenanordnung basierendes Verfahren zur Auswahl einer physiologisch standardisierten Ableitung beschrieben. Diese standardisierte Ableitung wird aus den größten Beträgen der P-. Q-, R-. S- und T-Welle berechnet.

Eine Verknüpfung von zwei oder mehr Ableitungskanälen zur Verbesserung des Rauschabstands ist bei sogenannten Holter-EKG-Anordnungen vorgesehen, wie sie beispielsweise in Chr. Zywietz, W. Grabbe, G. Hempel, "HES LKG, A New Program for Computer Assisted Analysis of Holter Electrocardiograms", in Computers in Cardiology, Florenz, Italien, S. 169 - 172, 23.-25. September 1981, oder in US 4,860,762 beschrieben sind. Bei diesen Holter-EKG-Anordnungen werden gestörte Kanäle abgeschaltet und Kombinationen der Signale der verbleibenden Kanäle weiter verarbeitete. Bei k Kanälen ergeben sich so 2^{k}-1 verschiedene Kombinationen was bei 15 Kanälen 32767 mögliche Kombinationen ergibt. Der Rechenaufwand ist entsprechend groß und aufwändig, was die für die Auswertung notwendige Zeit verlängert.

Bei einem Belastungs-Elektrokardiogramm treten die meisten Störungen bekanntlich in der Hochbelastungsphase auf. Die Hochbelastungsphase ist ihrerseits aber eine der wichtigsten Phasen eines Belastungstests. So entscheidet der Arzt unter anderem während dieser Phase, ob die Untersuchung abgebrochen werden soll oder nicht.

Zur Entscheidung, ob die Hochbelastungsphase abgebrochen werden soll oder nicht, werden unter anderem die Herzfrequenz, die ST-Strecke des Elektrokardiogramms und Arrhythmieereignisse herangezogen.

Es ist daher von großer Bedeutung, daß gerade in der Hochbelastungsphase trotz der häufiger auftretenden Störungen die für die Entscheidung relevanten Daten richtig und schnell berechnet werden können. Voraussetzung für eine solche richtige Berechnung der Daten ist aber eine genaue Lokalisation der QRS-Komplexe.

Es ist daher Aufgabe der vorliegenden Erfindung, eine Elektrokardiogramm-Anordnung zu schaffen, die auch während Hochbelastungsphasen eine genaue Lokalisation von QRS-Komplexen erlaubt.

Diese Aufgabe wird bei einer Elektrokardiogramm-Anordnung der eingangs genannten Art erfindungsgemäß dadurch gelöst, daß
- in den mehreren Ableitungskanälen n (2 < n ≤ m) unabhängige Auswerteeinheiten vorgesehen sind, wobei jedem Ableitungskanal jeweils höchstens eine Auswerteeinheit zugeordnet ist, die eine QRS-Komplex-Lokalisation, eine Ereignis-Klassifizierung sowie eine EKG-Qualitätsbestimmung vornimmt.
- ein Vergleicher in der mit den einzelnen Auswerteeinheiten verbundenen zentralen Logikeinheit die durch die EKG-Qualitätsbestimmungen in den einzelnen Ableitungskanälen gewonnenen EKG-Qualitäten miteinander vergleicht und den Ableitungskanal, dem die höchste EKG-Qualität zukommt, als dominanten Ableitungskanal ermittelt, und
- eine Prüfeinheit in der zentralen Logikeinheit, die Ergebnisse der Ereignis-Klassifizierung des dominanten Ableitungskanals überprüft und bei Auftreten besonderer Ereignisse im dominanten Ableitungskanal auf andere Ableitungskanäle zugreift, um davon abhängig die Ergebnisse der QRS-Komplex-Lokalisation und der Ereignis-Klassifizierung des dominanten Ableitungskanals zu korrigieren.

Die erfindungsgemäße Elektrokardiogramm-Anordnung nutzt dabei zunächst die Erkenntnis aus, daß während einer Hochbelastungsphase die Signale von nahezu allen Ableitungskanälen gestört sind. Es ist nämlich oft der Fall, daß nur die Signale von einem oder von zwei Ableitungskanälen verwertbar sind, während die Signale von allen anderen Ableitungskanälen die Ergebnisse verfälschen, da sie zu stark gestört sind.

Bei der erfindungsgemäßen Elektrokardiogramm-Anordnung werden die QRS-Komplexe in den verschiedenen Ableitungskanälen separat ausgewertet und lokalisiert. Das heißt, es wird im Gegensatz zum Stand der Technik auf eine Zusammenfassung der einzelnen EKG-Kurven der verschiedenen Ableitungskanäle zu einem Signal verzichtet. Vielmehr werden in jedem einzelnen Ableitungskanal Qualitätsbestimmungen vorgenommen und hierzu Qualitätsmerkmale separat berechnet. Die Zeitpunkte der so lokalisierten QRS-Komplexe und deren Ereignis-Klassifizierung werden von dem Ableitungskanal mit der höchsten EKG-Qualität, dem sogenannten dominanten Ableitungskanal übernommen und beispielsweise für die Berechnung der Herzfrequenz verwendet.

Bei bestimmten Ereignissen im Elektrokardiogramm des dominanten Ableitungskanales, wie beispielsweise einer Pause, einer supraventrikulären Extrasystole oder eines Fusionsschlages, wird auf die Merkmale der QRS-Komplexe aus anderen Ableitungskanälen zugegriffen. Tritt beispielsweise eine Pause im dominanten Ableitungskanal auf, so werden die entsprechenden Merkmale eines anderen Ableitungskanales übernommen, wenn dort ein QRS-Komplex, beispielsweise eine ventrikuläre Extrasystole, lokalisiert wurde.

Merkmale für die EKG-Qualitätsbestimmung sind beispielsweise:
- die Amplitude der QRS-Komplexe.
- ein Störgradwert für hochfrequente Störungen, die beispielsweise durch
   Muskelzittern entstehen.
- ein Störgradwert für mittelfrequente Störungen, die beispielsweise durch Elektrodenbewegungen relativ zum Körper des Patienten entstehen, und
- der Elektrodenzustand, wie beispielsweise abgefallene Elektroden.

Wesentlich an der erfindungsgemäßen Elektrokardiogramm-Anordnung zur zuverlässigen Lokalisation von QRS-Komplexen in einem Elektrokardiogramm sind also insbesondere die folgenden Merkmale:
- Unabhängige Auswerteeinheiten, die äquivalent zueinander sind, dienen zur Lokalisation und Ereignis-Klassifizierung von QRS-Komplexen und zur EKG-Qualitätsbestimmung in jedem einzelnen Ableitungskanal.
- Eine zentrale Logikeinheit übernimmt, abhängig von der EKG-Qualität der einzelnen Ableitungskanäle, die Ergebnisse der QRS-Lokalisation und der Ereignis-Klassifizierung von dem "besten" Ableitungskanal.
- Die zentrale Logikeinheit überprüft die Ereignis-Klassifizierung des "besten" Ableitungskanals auf Ereignisse, wie beispielsweise Pausen, supraventrikuläre Extrasystolen oder Fusionsschläge, greift auf die Ergebnisse der anderen Ableitungskanäle zu und korrigiert abhängig von deren Ergebnissen die Ergebnisse der Ereignis-Klassifizierung und der QRS-Lokalisation des "besten" bzw. dominanten Ableitungskanals.

Durch die auf diese Weise vorgenommene spezifische Auswahl der Ableitungskanale mit den am wenigstens gestörten Signalen wird eine Verbesserung der QRS-Komplex-Lokalisation gegenüber herkömmlichen Anordnungen erreicht. Als Folge davon wird die Qualität der darauf aufbauenden Algorithmen, wie Herzfrequenzermittlung, und sogar Mittelwertschlagsbildung für die ST-Vermessung verbessert.

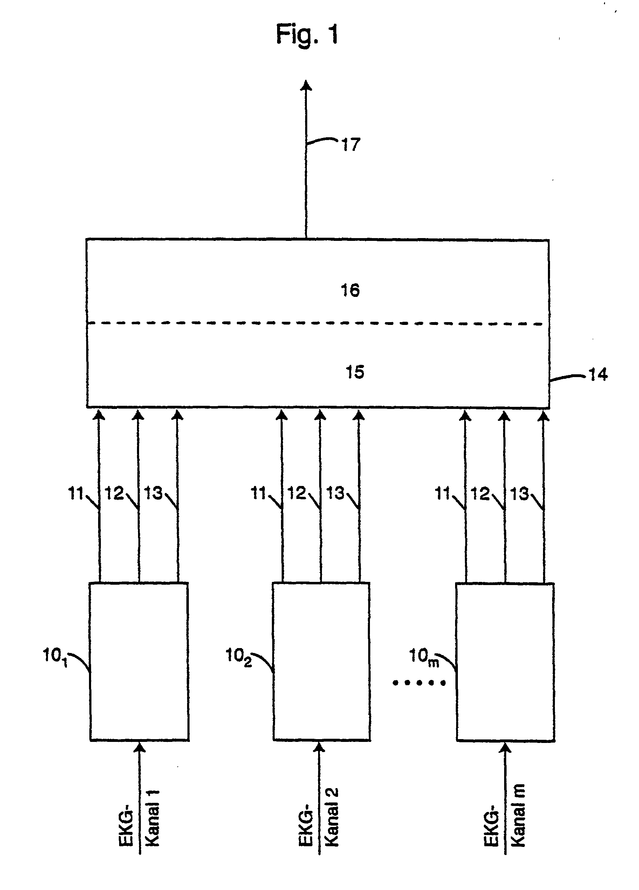

Nachfolgend wird die Erfindung anhand der Zeichnung naher erläutert, in deren einziger Figur ein Blockschaltbild der erfindungsgemäßen Elektrokardiogramm-Anordnung schematisch dargestellt ist.

Bei der erfindungsgemäßen Elektrokardiogramm-Anordnung sind EKG-Ableitungskanäle 1, 2, ..., m Auswerteeinheiten 10₁, 10₂, ..., 10ₘ zugeführt, wobei jedoch nicht unbedingt jeder EKG-Ableitungskanal eine solche Auswerteeinheit 10₁, 10₂, ..., 10ₘ aufzuweisen braucht. Jedoch ist in jedem EKG-Ableitungskanal nicht mehr als eine Auswerteeinheit vorgesehen, so daß die Anzahl der Auswerteeinheiten 10₁, 10₂, ..., 10ₘ höchstens der Anzahl der EKG-Ableitungskanäle entspricht. In diesen EKG-Auswerteeinheiten wird jeweils eine QRS-Komplex-Lokalisation, eine Ereignis-Klassifizierung sowie eine EKG-Qualitätsbestimmung vorgenommen, was in üblicher Weise geschieht. Die Ergebnisse dieser QRS-Komplex-Lokalisation, dieser Ereignis-Klassifizierung und dieser EKG-Qualitätsbestimmung werden jeweils, wie dies durch Pfeile 11, 12 bzw. 13 angedeutet ist, einer zentralen Logikeinheit 14 zugeführt, die insbesondere einen Vergleicher 15 und eine Prüfeinheit 16 aufweist. Der Vergleicher 15 vergleicht die mit den einzelnen Auswerteeinheiten 10₁, 10₂, ..., 10ₘ für die Ableitungskanäle gewonnenen EKG-Qualitäten miteinander und ermittelt den Ableitungskanal, dem die höchste EKG-Qualität zukommt. Die QRS-Komplex-Lokalisation (vergleiche die Pfeile 11) des Ableitungskanals, der die höchste EKG-Qualität hat, wird sodann von dem Vergleicher 15 für eine Weiterverarbeitung von diesem ausgewählt. Die Prüfeinheit 16 überprüft die Ereignis-Klassifizierung des dominanten Ableitungskanals und greift bei Auftreten besonderer Ereignisse, wie beispielsweise Pausen, supraventrikulärer Extrasystolen und Fusionsschläge, auf andere Ableitungskanäle zu und korigiert abhängig von diesem Zugriff die Ereignis-Klassifizierung und das Ergebnis der QRS-Komplex-Lokalisation des dominanten Ableitungskanals.

Für die genannte Weiterverarbeitung kommt beispielsweise die Ermittlung der Herzfrequenz in Betracht. Es sind aber auch andere Weiterverarbeitungen möglich.

Schließlich wird von der zentralen Logikeinheit 14 das QRS-Lokalisationsergebnis und das Ereignis-Klassifizierungsergebnis (vergleiche Pfeil 17) ausgegeben.

## Patentansprüche

1. Elektrokardiogramm-(EKG-)Anordnung mit m (m > 2) Ableitungskanälen (1, 2, ..., m) zur Lokalisation von QRS-Komplexen in einem Elektrokardiogramm und mit einer zentralen Logikeinheit (14),
**dadurch gekennzeichnet, daß**
- in den mehreren Ableitungskanälen (1, 2, ..., m) n (2 < n ≤ m) unabhängige Auswerteeinheiten (10₁, 10₂, ...) vorgesehen sind, wobei jedem Ableitungskanal jeweils höchstens eine Auswerteeinheit (10₁, 10₂, ...) zugeordnet ist, die eine QRS-Komplex-Lokalisation (11), eine Ereignis-Klassifizierung (12) sowie eine EKG-Qualitätsbestimmung (13) vornimmt,
- ein Vergleicher (15) in der mit den einzelnen Auswerteeinheiten (1, 2, ...) verbundenen zentralen Logikeinheit (14) die durch die EKG-Qualitätsbestimmungen in den einzelnen Ableitungskanälen (1, 2, ...) gewonnenen EKG-Qualitäten miteinander vergleicht und den Ableitungskanal, dem die höchste EKG-Qualität zukommt, als dominanten Ableitungskanal ermittelt, und
- eine Prüfeinheit (16) in der zentralen Logikeinheit (14), die Ergebnisse der Ereignis-Klassifizierung (12) des dominanten Ableitungskanals überprüft und bei Auftreten besonderer Ereignisse im dominanten Ableitungskanal auf andere Ableitungskanäle zugreift, um davon abhängig die Ergebnisse der QRS-Komplex-Lokalisation (11) und der Ereignis-Klassifizierung (12) des dominanten Ableitungskanals zu korrigieren.

2. Anordnung nach Anspruch 1, **dadurch gekennzeichnet, daß** die besonderen Ereignisse Pausen, supraventrikuläre Extrasystolen und Fusionsschläge sind.

3. Anordnung nach Anspruch 1 oder 2, **dadurch gekennzeichnet, daß** bei der Weiterverarbeitung die Herzfrequenz ermittelt wird.

## Claims

1. Electrocardiogram (ECG) arrangement having m (m > 2) lead channels (1, 2, ..., m) for locating QRS complexes in an electrocardiogram and having a central logic unit (14),
**wherein**
- n (2 < n ≤ m) independent evaluation units (10₁, 10₂, ...) are provided in the plurlity of lead channels (1, 2, ..., m), each lead channel being respectively assigned at most one evaluation unit (10₁, 10₂, ...) which locates the QRS complexes (11), classifies the events (12) and assesses the ECG quality (13),
- a comparator (15), in the central logic unit (14) connected to the individual evaluation units (1, 2, ...), which compares with one another the ECG qualities obtained by the ECG quality assessments in the individual lead channels (1, 2, ...) and identifies the lead channel having the highest ECG quality as the dominant lead channel, and
- a test unit (16), in the central logic unit (14), which tests the results of the event classification (12) of the dominant lead channel and, when special events occur in the dominant lead channel, accesses other lead channels in order to correct the results of the QRS complex location (11) and the event classification (12) of the dominant lead channel on that basis.

2. Arrangement as claimed in claim 1, **wherein** special events are pauses, superventricular extrasystoles and fusion beats.

3. Arrangment as claimed in claim 1 or 2, wherein the heart rate is determined in the subsequent processing.

## Revendications

1. Dispositif d'électrocardiographie (ECG) avec m (m > 2) canaux de dérivation (1, 2, ..., m) pour la localisation de complexes QRS dans un électrocardiogramme et avec une unité logique centrale (14), **caractérisé en ce que**
- dans la pluralité de canaux de dérivation (1, 2, ..., m) sont prévues n (2 < n ≤ m) unités d'analyse indépendantes (10₁, 10₂, ...), à chacun des canaux de dérivation étant associée au plus une unité d'analyse (10₁, 10₂, ...) qui procède à une localisation des complexes QRS (11), à une classification des événements (12) ainsi qu'à une analyse qualitative d'ECG (13),
- un comparateur (15) dans l'unité logique centrale (14) connectée aux unités d'analyse individuelles (1, 2, ...) compare entre elles les qualités d'ECG recueillies par l'intermédiaire des analyses qualitatives d'ECG et désigne comme canal de dérivation dominant le canal de dérivation auquel correspond la qualité d'ECG la plus élevée, et
- une unité de contrôle (16) dans l'unité logique centrale (14), laquelle contrôle les résultats de la classification des événements (12) du canal de dérivation dominant et, en présence d'événements particuliers dans le canal de dérivation dominant, fait appel à d'autres canaux de dérivation afin de corriger, en fonction de ces derniers, les résultats de la localisation des complexes QRS (11) et de la classification des événements (12) du canal de dérivation dominant.

2. Dispositif selon la revendication 1, **caractérisé en ce que** les événements particuliers sont des pauses, des extrasystoles supraventriculaires et des battements de fusion.

3. Dispositif selon la revendication 1 ou 2, **caractérisé en ce que**, lors du traitement ultérieur, la fréquence cardiaque est déterminée.
